# EUROPEAN PATENT SPECIFICATION

(11) **EP 2 275 063 B1**
(45) Date of publication and mention of the grant of the patent: **01.10.2014**
(21) Application number: 09726648.0
(22) Date of filing: 31.03.2009
(51) Int. Cl.: A61F 13/15, A61F 13/49, A61F 13/56

(54) **METHOD OF MANUFACTURING ABSORPTIVE ARTICLE AND DEVICE FOR MANUFACTURING ABSORPTIVE ARTICLE**
VERFAHREN ZUR HERSTELLUNG VON SAUGFÄHIGEN TEILCHEN UND VORRICHTUNG ZUR HERSTELLUNG VON SAUGFÄHIGEN TEILCHEN
PROCÉDÉ DE FABRICATION D'UN ARTICLE ABSORBANT ET DISPOSITIF DE FABRICATION D'UN ARTICLE ABSORBANT

(30) Priority: 31.03.2008 JP 2008094119; 25.03.2009 JP 2009074763
(43) Date of publication of application: 19.01.2011
(73) Proprietor: Unicharm Corporation, Ehime 799-0111 (JP)
(72) Inventor: SAKAGUCHI, Satoru, Kanonji-shi Kagawa 769-1602 (JP)
(74) Representative: Eke, Philippa Dianne
(86) International application number: PCT/JP2009/056631
(87) International publication number: WO 2009/123182

(56) References cited:
- WO-A1-84/04242
- WO-A1-96/03952
- WO-A1-03/057116
- JP-A- 48 049 542
- JP-A- 2006 247 364
- JP-T- 2005 503 892
- JP-T- 2005 533 529
- JP-U- 59 165 404
- US-A1- 2004 188 004

## Description

### TECHNICAL FIELD

The present invention relates to a manufacturing method and a manufacturing apparatus for absorbent articles each of which includes: a front waistline member, a rear waistline member, and a crotch member that connects the front waistline member and the rear waistline member.

### BACKGROUND ART

In general, in order to be easily put on the wearer, an open-type diaper needs to be provided with: a vertically long absorber (absorber body) that absorbs body fluid from the body of the wearer, a diaper main body to which the absorber is attached, side flaps (hereinbelow referred to as flaps) that project outwardly in the width direction of the absorber from the absorber body.

Heretofore, as a manufacturing method for such an open-type diaper provided with flaps, the following method has been known (Patent Literature 1, for example).
(1) forming, on a continuum of flaps (continuous belt part), a potential cut line between each first belt part sheet fastener and each second belt part sheet fastener both placed in a crossing direction (CD direction) that is orthogonal to the moving direction (MD direction) of the continuum of flaps (flap continuum).
(2) placing the continuum of flaps (continuous belt part) along one edge portion of a continuum of the diaper main bodies (diaper main body continuum), and forming a bonding region in the CD direction.
Patent Literature 1: Japanese Patent Laid-open Publication No. 2006-340862

However, in the above manufacturing method for the open-type diaper, the potential cut line is orthogonal to the MD direction (moving direction) of a flap line on which the continuum of flaps (continuous belt part) is transported. Accordingly, there has been a problem that, when the strength of the potential cut line is increased so as to prevent the continuum of flaps from being torn along the potential cut line on the flap line, a user of the manufactured diaper may find it difficult to tear along the potential cut line.

Further, in the above manufacturing method for the open-type diaper, the potential cut line is orthogonal to the MD direction of the flap line on which the flap continuum (continuous belt part) is transported. Accordingly, when the strength of the potential cut line is formed to be weak so that a user can easily tear along it, the potential cut line may be torn while the flap continuum is continuously transported on the flap line. Thus, there has been a problem that the flap continuum cannot be stably transported on the flap line, or a manufacturing facility for the diapers becomes complex since high level controls are required for continuously transporting the flap continuum.

US 2004/0188004 A1 also relates to a method of applying a fastener portion to a diaper.

The present invention has been made in view of the above-described problems, and has an object of providing a manufacturing method and a manufacturing apparatus for absorbent articles being able to form side flaps that can be easily unfolded and developed by the user of the manufactured diaper, and being able to achieve a continuous transportation of a flap continuum on a flap line without using high level controls.

### DISCLOSURE OF THE INVENTION

An aspect of the invention is summarized as a manufacturing method for absorbent articles each including a front waistline member, a rear waistline member, and a crotch member, the crotch member connecting the front waistline member and the rear waistline member. The manufacturing method for the absorbent articles includes: placing latch members that can be attached to and locked in predetermined regions, on both sides of a vicinal region of a center line extending along a moving direction of a flap line or a direction crossing the moving direction of the flap line, on an area each constituting flaps from a flap continuum having a long shape and being continuously transported on a flap line; forming a potential cut line along the center line; bonding, on a main body line, a front waistline member continuum or a rear waistline member continuum onto the flap continuum or onto the flaps, so that the center line is orthogonal to a moving direction of the main body line; the flaps being formed by cutting the flap continuum along a direction crossing the moving direction of the flap line; and tearing along the potential cut line on the main body line.

As described above, the present invention can provide a manufacturing method and a manufacturing apparatus for absorbent articles being able to form side flaps that can be easily unfolded and developed by the user of the manufactured diapers, and being able to achieve a continuous transportation of a flap continuum on a flap line without using high level controls.

### BRIEF DESCRIPTION OF DRAWINGS

[Fig. 1] Fig. 1 is a diagram illustrating a manufacturing method for absorbent articles according to a first embodiment of the present invention.
[Fig. 2] Fig. 2 is a diagram illustrating a potential cut line cutting step in a manufacturing method for absorbent articles according to the first embodiment of the present invention.
[Fig. 3] Fig. 3A and 3B are plan views of absorbent articles according to the first embodiment of the present invention
[Fig. 4] Fig. 4 is a diagram for illustrating a potential cut line cutting step in a manufacturing method for absorbent articles according to a modified example 1 of the present invention.
[Fig. 5] Fig. 5 is a diagram illustrating a manufacturing method for absorbent articles according to a modified example 2 of the present invention.
[Fig. 6] Fig. 6 is a diagram illustrating a manufacturing method for absorbent articles according to a modified example 3 of the present invention.
[Fig. 7] Fig. 7 is a diagram illustrating a manufacturing method for absorbent articles according to a modified example 4 of the present invention.
[Fig. 8] Fig. 8 is a perspective view of a latch member placing structure 310 according to the second embodiment of the present invention.
[Fig. 9] Fig. 9 is a perspective view of a potential cut line forming structure 320 according to the second embodiment of the present invention.
[Fig. 10] Fig. 10 is a perspective view of a bonding structure 330 according to the second embodiment of the present invention.

### PREFERRED EMBODIMENTS FOR CARRYING OUT THE INVENTION

### [First Embodiment]

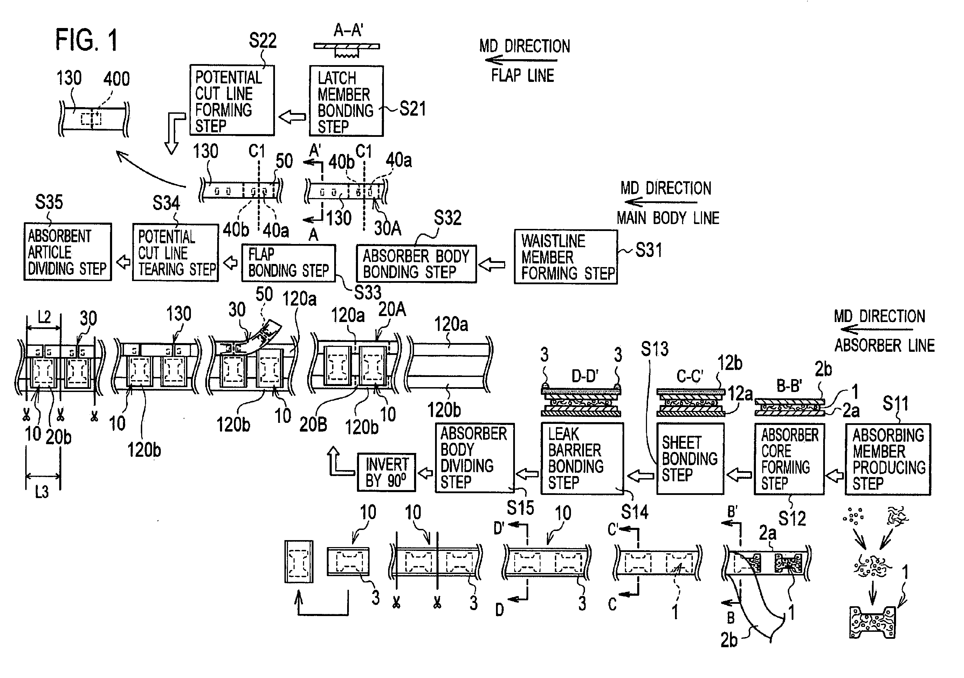

Description will be given of a manufacturing method for absorbent articles according to a first embodiment of the present invention with reference to Figs. 1 to 3.

According to the manufacturing method for absorbent articles of the first embodiment, absorbent articles, each of which includes a front waistline member 20b, a rear waistline member 20a, and a crotch member (absorber body) 10 connecting the front waistline member 20b and the rear waistline member 20a can be manufactured (see, Fig. 1 to Fig. 3).

Hereinafter, with reference to Fig. 1, description will be given of a manufacturing method for absorbent articles according to this embodiment.

Firstly, steps performed on the absorber line, on which the absorbers placed on the crotch members 10 are transported, will be described.

In step S11, a crusher crushes a pulp sheet to produce crushed pulp. Thereafter, when the crushed pulp passes through a pulp supply duct, a super absorbent polymer (SAP) is mixed thereto.

Then, the mixture of the crushed pulp and the super absorbent polymer, which is supplied from the pulp supply duct, is layered in a predetermined shape by use of a layer drum, thereby absorber cores 1 (absorbers) are formed.

In step S12, on sheet tissues 2a being continuously transported in the moving direction (hereinafter referred to as MD direction) of the absorber line, the absorber cores 1 are respectively placed by a predetermined interval length in the MD direction (moving direction) of the absorber line.

Subsequently, on the sheet tissues 2a, sheet tissues 2b being continuously transported in the MD direction of the absorber line are layered. Here, on the sheet tissues 2a, each of the absorber cores 1 is placed along the MD line of the absorber line.

In step S13, top sheets (liquid permeable) 12b being continuously transported in the MD direction of the absorber line are respectively placed on and bonded with the sheet tissues 2b. In addition, back sheets (liquid impermeable) 12a being continuously transported in the MD direction of the absorber line are respectively placed under and bonded with the sheet tissues 2a.

In step S14, three-dimensional gathers 3 formed of different members are bonded onto both side edges of each of the top sheets 12b in the MD direction of the absorber line.

In step S15, a continuum of crotch members (a crotch member continuum 110) (absorber body) that is continuously transported in the MD direction of the absorber line is transported by use of a rotation of a rotating drum. Concurrently, the crotch member continuum 110 is cut in the CD direction by a predetermined interval length, by use of a cutter roll arranged opposite to the outer peripheral surface of the rotating drum. Thereby each of the crotch members 10 is formed.

Secondly, steps performed on the flap line, on which the flaps 30 to be placed on the rear waistline members 20a are transported, will be described.

In step S21, in a region 30A each constituting one flap on the flap continuum 130 having a long shape and being continuously transported on the flap line, latch members 40a and 40b that can be attached to and locked in predetermined regions (for example, predetermined regions in the front waistline members 20b) are placed on both sides of the center line C1 that extends along the CD direction of the flap line.

In this regard, when hook members (male members) are placed as the latch members 40a and 40b, loop members (female members) are provided as latched members in the predetermined regions of the front waistline members 20b.

Note that, in the present embodiment, the front waistline members 20b are made of a non-woven fabric. Accordingly, the predetermined regions of the front waistline members 20b themselves can serve as the loop members even when the loop members are not additionally provided.

In addition, one latch member may be placed across both sides of the center line C1 instead of respectively placing the two latch members 40a and 40b on both sides of the center line C1.

In step S22, on the flap continuum 130, a potential cut line (perforated line) 50 that halves the region 30A is formed.

Note that, the potential cut line 50 does not need to be consistent with the center line C1. The potential cut line 50 may be straight or curved as long as it extends along the center line C1.

Furthermore, steps S21 and S22 described above may be performed in reverse order. To be more specific, in step S22, the latch members 40a and 40b may be placed on both sides of the center line C1, in the region 30A each constituting one flap from the flap continuum 130, after forming the potential cut line (perforated line) 50.

Thirdly, steps performed on the main body line, on which the front waistline member continuum 120b and the rear waistline member continuum 120a are transported, will be described.

In step S31, a pair of continuums of waistline members (the front waistline member continuum 120b and the rear waistline member continuum 120a) both having a long shape is produced from non-woven fabric and is transported in the MD direction of the main body line.

Note that, the front waistline member continuum 120b and the rear waistline member continuum 120a may have stretching properties.

In step S32, the crotch members 10 each formed in step S15 on the absorber line are placed while being spaced apart from one another in the MD direction of the main body line, between the front waistline member continuum120b and the rear waistline member continuum 120a. Here, both of the front waistline member continuum120b and the rear waistline member continuum 120a have a long shape and are continuously transported on the main body line.

In step S33, on the main body line, the flap continuum 130 is bonded onto the rear waistline member continuum 120a so that each of the regions 30A constituting each of the flaps 30 on the flap continuum 130 respectively corresponds to each of the regions 20A constituting each of the absorbent articles (rear waistline members 20a).

In other words, the flap continuum 130 is bonded to the rear waistline member continuum 120a so that the latch members 40a and 40b can be placed in the vicinity of the center area of the rear waistline members 20a in the absorbent articles.

In this regard, in step S33, it is preferable that the regions 30A each constituting each of the flaps 30 on the flap continuum 130 is bonded with the region 20A each constituting each of the absorbent articles (rear waistline members 20a), along the edge portion in the width direction of the absorbent articles.

In this regard, in step S33, it is preferable that the flap continuum 130 be bonded with the rear waistline members 20a by use of a thermo compression or the like, using an embossing roller or ultrasonic waves. Further, thermoplastic resin may be applied along the edge portions A1 and A2 of the flaps 30. Furthermore, thermoplastic resin may be entirely applied onto the back side of the flap continuum 130, or thermoplastic resin may be applied onto only a part of the back side of the flap continuum 130.

Moreover, in step S33, the latch members 40a and 40b may be placed on a pair of folded back portions respectively, and may be attached to and locked on the rear waistline member continuum 120a.

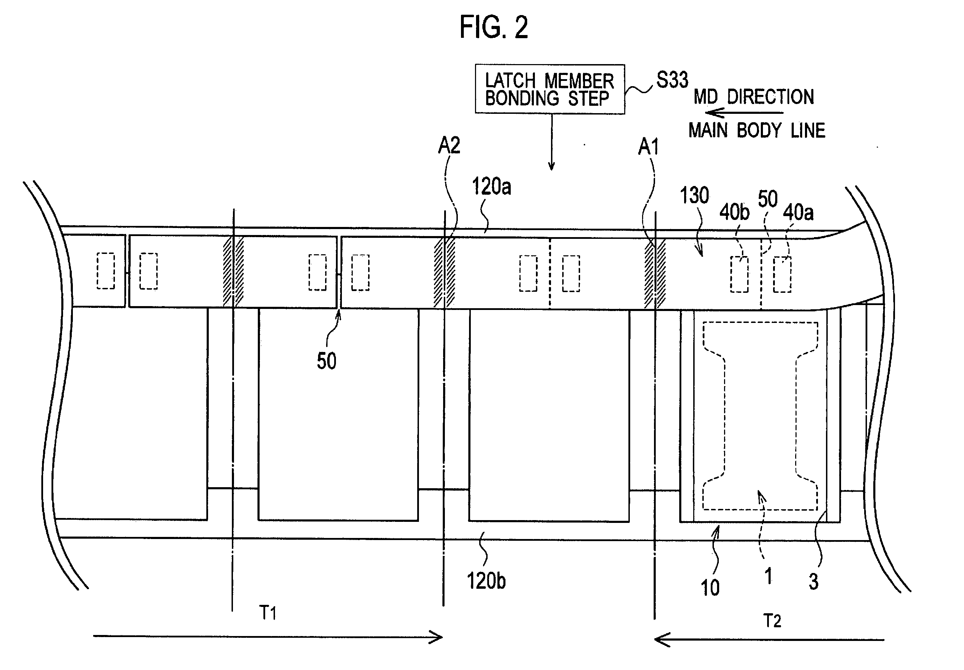

In step S34, on the main body line, the potential cut line 50 is torn. Specifically, as shown in Fig. 2, the flap continuum is torn along the potential cut line 50 by setting a tension T1 stronger than a tension T2. Here, the tension T1 is employed in the steps subsequent to the flap bonding step S33 (bonding step) on the main body line while the tension T2 is employed in the flap bonding step S33 (bonding step) and the previous steps.

In step S35, on the main body line, the front waistline member continuum 120b and the rear waistline member continuum 120a are cut in the CD direction of the main body line by a predetermined interval length.

Specifically, the front waistline member continuum 120b and the rear waistline member continuum 120b are cut so that a length L3 of each of the front waistline members 20b in the width direction of the absorbent articles and a length L2 of each of the rear waistline members 20a in the width direction of the absorbent articles can be equal.

Note that, in the above-described example, description has been given of the example in which the flap continuum 130, on which the latch members 40a and 40b that can be attached to and locked in the predetermined regions of the front waistline members 20b are placed, is placed on the rear waistline member continuum 120a. However, the present invention is not limited to this example but can be applied to an example in which the flap continuum 130, on which the latch members 40a and 40b that can be attached to and locked in the predetermined regions of the front waistline members 20b are placed, is placed on the front waistline member continuum 120b.

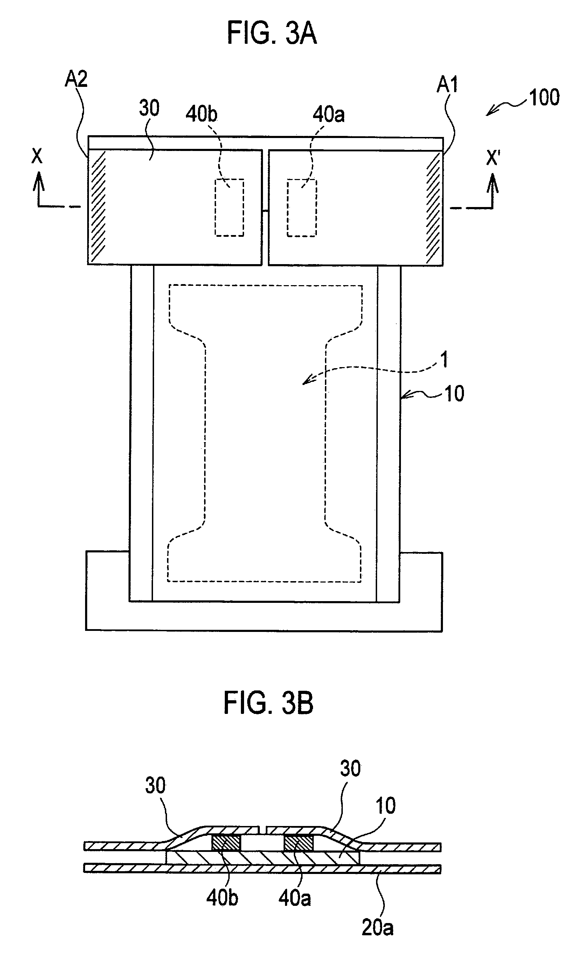

Figs. 3A and 3B show an overall structure of an open-type diaper (absorbent article) manufactured by the manufacturing method for absorbent articles according to this embodiment.

As shown in Figs. 3A and 3B, in the open-type diaper manufactured by the manufacturing method for absorbent articles according to this embodiment, the potential cut line 50 is torn and the latch members 40a and 40b are attached to and temporarily bonded to the rear waistline members 20a.

According to the manufacturing method for absorbent articles according to the first embodiment of the present invention, it is possible to form the side flaps that can be easily unfolded and developed by the user, while achieving a continuous transportation of the flap continuum 130 on the flap line without using high level controls.

Specifically, in the manufacturing method for absorbent articles according to the first embodiment of the present invention, the flap continuum can be torn along the potential cut lines 50 on the main body line. Accordingly, this method enables simultaneous achievement of two objects that are difficult to attain at the same time, that is, to be capable of continuously transporting a flap continuum on a flap line, and to manufacture diapers having potential cut lines along which the users can easily tear.

According to the manufacturing method for the absorbent article of the first embodiment of the present invention, the front waistline member continuum 120b and the rear waistline member continuum 120a are continuously transported. Accordingly, the edge portion of the front waistline member continuum 120b and the edge portion of the rear waistline member continuum 120a can be easily folded back and stacked. Therefore, the front waistline member continuum 120b and the rear waistline member continuum 120a thus stacked can be simultaneously cut. Thus, an open-type diaper and a pants-type diaper can be manufactured by the same cutting step. Therefore, the open-type diapers and the pants-type diapers can be manufactured without changing the cutting step or a cutter itself.

### (Modified example 1)

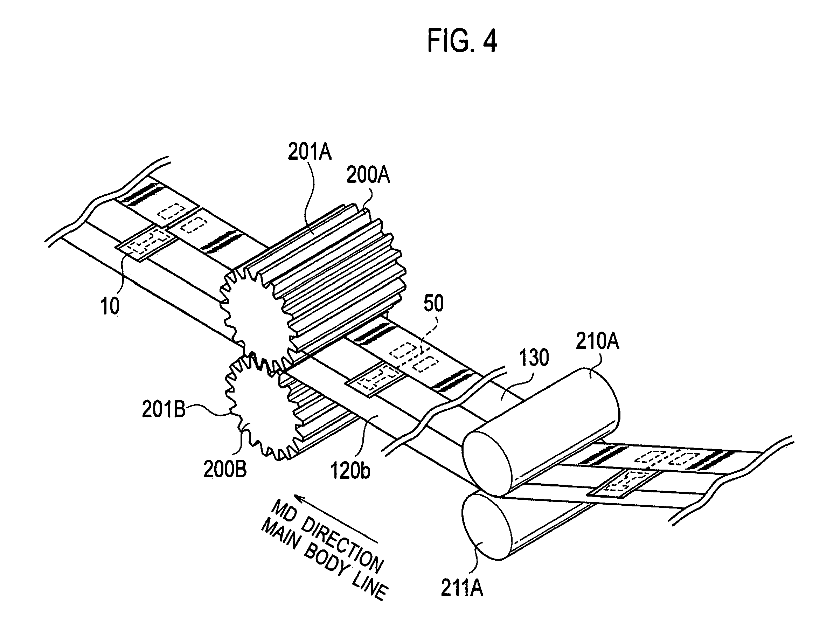

With reference to Fig. 4, description will be given of a manufacturing method for absorbent articles according to a modified example 1 of the first embodiment of the present invention. Hereinafter, the manufacturing method for absorbent articles according to the modified example 1 of the present invention will be described, mainly focusing on the differences from the manufacturing method for absorbent articles according to the first embodiment of the present invention.

As shown in Fig. 4, in the manufacturing method for absorbent articles according to the modified example 1, in step S34, the flap continuum is torn along the aforementioned potential cut line 50, on the main body line by pressing the potential cut line 50 with a tearing structure 200.

The tearing structure 200 includes a pair of rollers 200A and 200B. The roller 200A has a protruding portion 201 A. On the other hand, the roller 200B includes a protruding portion 201B that engages with the protruding portion 201 A.

Note that, in this manufacturing method for absorbent articles according to the modified example 1, in step S34, the pair of roller 200A and 200B is not necessarily provided. Alternatively, the aforementioned potential cut lines 50 may be torn by performing a hammer press processing.

In this regard, in step S34, a region including the aforementioned potential cut line 50 may be heat treated to be made into films. For example, it is assumed that the flap continuum 130 is formed of polyethylene. In this case, when the region including the potential cut line 50 in the flap continuum 130 is heat treated, fibers in the region lose their elasticity to become stiff. This makes it easier to tear the flap continuum along the potential cut line 50.

### (Modified example 2)

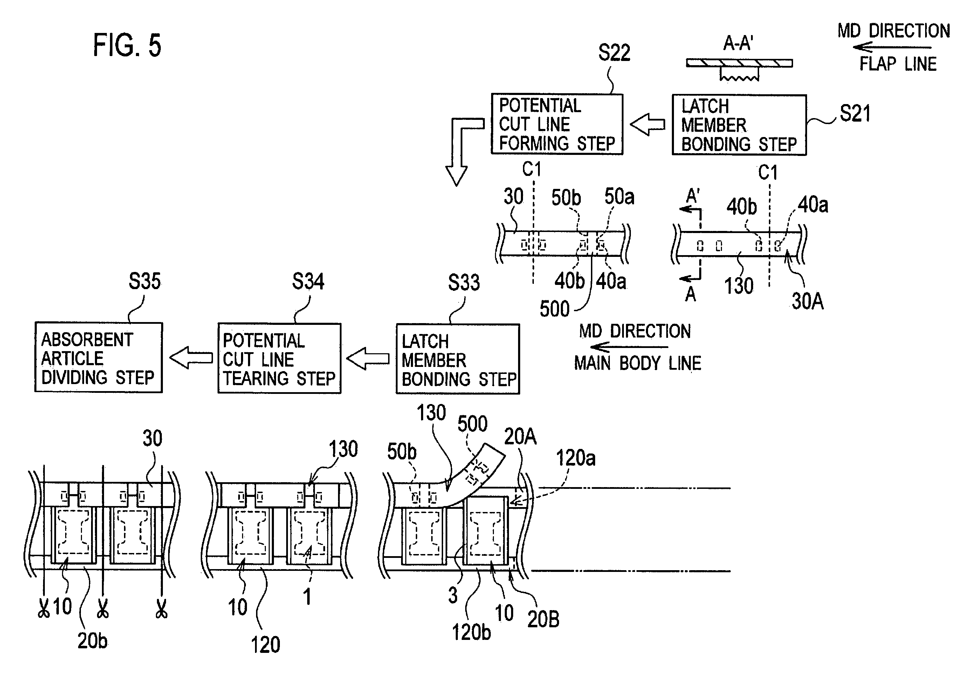

With reference to Fig. 5, description will be given of a manufacturing method for absorbent articles according to a modified example 2 of the present invention. Hereinafter, the manufacturing method for absorbent articles according to the modified example 2 of the present invention will be described mainly focusing on the differences from the manufacturing method for absorbent articles according to the first embodiment of the present invention.

As shown in Fig. 5, in step S22, on the flap line, two potential cut lines (perforated lines) 50a and 50b are formed along each center line C1 on the flap continuum 130.

Thereafter, in step S34, on the main body line, these two potential cut lines 50a and 50b are cut and a region 500 between these two potential cut lines 50a and 50b is removed.

### (Modified example 3)

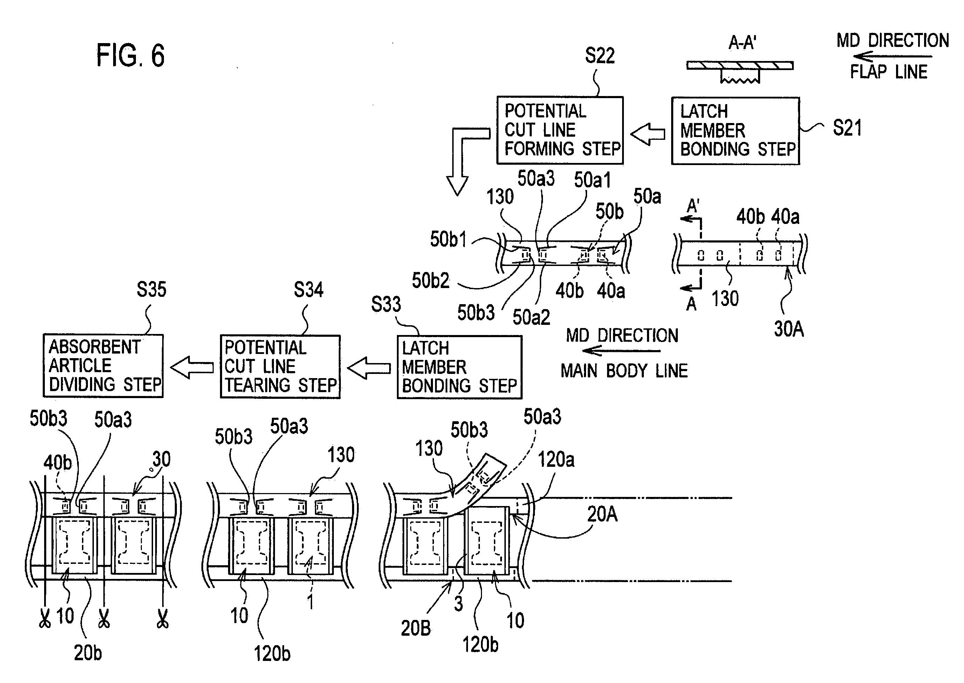

With reference to Fig. 6, description will be given of a manufacturing method for absorbent articles according to a modified example 3 of the present invention. Hereinafter, the manufacturing method for absorbent articles according to the modified example 3 of the present invention will be described mainly focusing on the differences from the manufacturing method for absorbent articles according to the first embodiment of the present invention.

As shown in Fig. 6, in step S22, on the flap line, two potential cut lines (perforated lines) 50a3 and 50b3 are formed along the center line C1 of the flap continuum 130.

Specifically, in step S22, in a part of the aforementioned region 30A, a pair of folded back portions 50a and 50b is formed in regions including the latch members 40a and 40b, respectively. Here, the folded back portion 50b can be folded in the MD direction of the flap line and in a direction opposite to the MD direction, respectively.

The folded back portion 50a is formed of a potential cut line 50a3 and two cut portions (slits) 50a1 and 50a2. The potential cut line 50a3 is formed to extend in the CD direction of the flap line while each of the cut portions 50a1 and 50a2 is formed to extend in the MD direction of the flap line.

Meanwhile, the folded back portion 50b is formed of a potential cut line 50b3 and two cut portions (slits) 50b1 and 50b2. The potential cut line 50b3 is formed to extend in the CD direction of the flap line while each of the cut portions 50b1 and 50b2 is formed to extend in the MD direction of the flap line.

Here, consider the case where a certain folded back portion 50b is folded back in the MD direction of the flap line and a folded back portion 50a, which is paired with the certain folded back portion 50b, is folded back in the direction opposite to the MD direction of the flap line, like hinges. Even in this case, the flap continuum 130 is not cut all along in the CD direction of the flap line, and thus any cut edge will not be folded back. Accordingly, the flap continuum 130 can be prevented from being turned over while being continuously transported.

Thereafter, in step S34, on the main body line, the two potential cut lines 50a3 and 50b3 are cut so that the aforementioned folded back portions 50a and 50b can be folded.

### (Modified example 4)

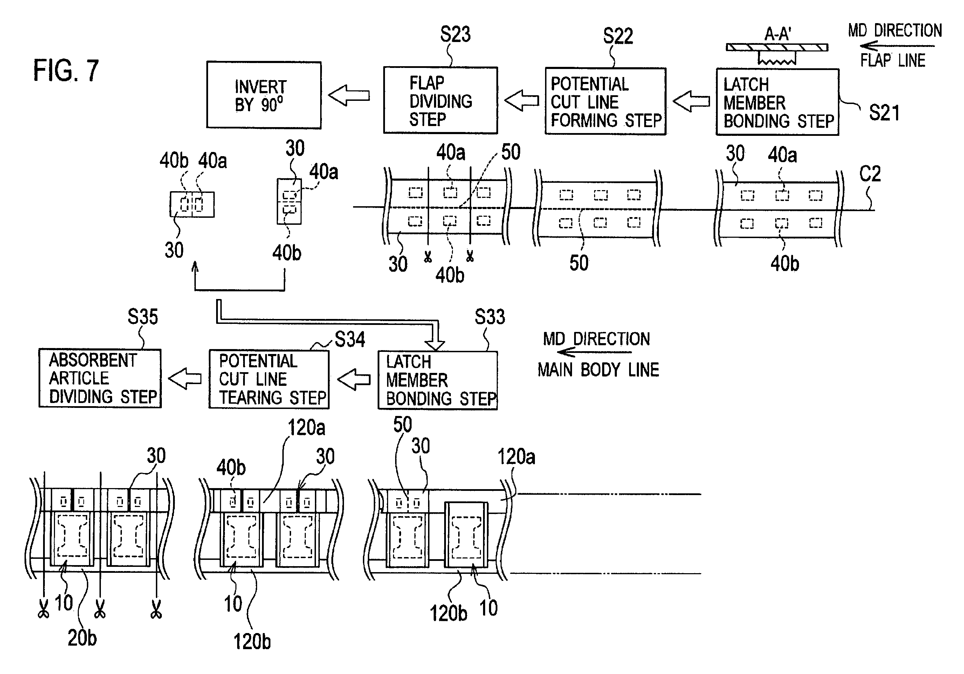

With reference to Fig. 7, description will be given of a manufacturing method for absorbent articles according to a modified example 4 of the present invention. Hereinafter, the manufacturing method for absorbent articles according to the modified example 4 of the present invention will be described mainly focusing on the differences from the manufacturing method for absorbent articles according to the first embodiment of the present invention.

As shown in Fig. 7, in step S21, on the flap continuum 130, latch members 40a and 40b that can be attached to and locked in the predetermined regions are placed on both sides of the center line C2 that extends in the MD direction of the flap line.

Specifically, on both sides of a vicinal region of the center line C2 of the flap continuum 130, the latch members 40a and 40b are bonded to the flap continuum 130, by a predetermined interval length.

In step S22, a potential cut line 50 is formed along the center line C2.

In step S23, on the flap line, the flap continuum 130 is cut in the CD direction of the flap line, and thereby each of the flaps 30 is formed.

In step S33, on the main body line, the flaps 30 formed in step S23 are placed on the rear waistline member continuum 120a so that the center line C2 crosses the MD direction of the main body line.

Specifically, the flaps 30 each formed in step S23 on the flap line is rotated by 90°, and thereafter is bonded onto the rear waistline member continuum 120a, by using a thermoplastic resin.

Here, it is preferable that the regions 20A each constituting the absorbent articles (rear waistline members 20a) on the rear waistline member 120a is bonded, along the edge portion in the width direction of the absorbent articles.

In this regard, in step S33, it is preferable that each of the flaps 30 be bonded with the rear waistline members 20a by use of a thermo compression or the like, using an embossing roller or ultrasonic waves. Furthermore, the thermoplastic resin may be applied along the edge portions A1 and A2 of the flaps.

### [Second Embodiment]

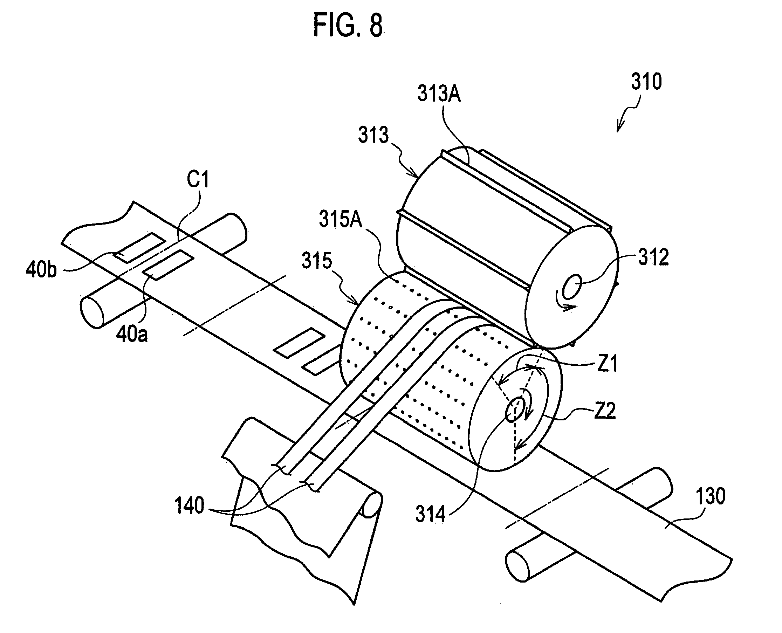

Hereinbelow, a manufacturing apparatus for absorbent articles according to the second embodiment will be described by referring to Figs. 8 to 10. The manufacturing apparatus for the absorbent article according to the second embodiment is provided with: a latch member placing structure 310; a potential cut line forming structure 320; and a tearing structure 200. Note that, the tearing structure 200 is same as that of the first embodiment, therefore the explanation thereof will be omitted.

First, the latch member placing structure 310 according to the second embodiment will be described by referring to the accompanying drawings. Fig. 8 is a perspective view of the latch member placing structure 310 according to the second embodiment of the present invention.

As shown in Fig. 8, as described in the above step S21, on the flap line, in a region 30A constituting one flap 30 from the flap continuum 130 having a long shape and being continuously transported, the latch member placing structure 310 places the latch members 40a and 40b that can be attached to and locked in predetermined regions, on both sides of the vicinal region of the center line C1 that extends along the MD direction of the flap line.

The latch member placing structure 310 includes: an upper blade roll 313 configured to rotate by using a shaft 312 as a center; and a lower blade roll 315 configured to rotate by using a shaft 314 as a center.

The upper blade roll 313 is provided with a plurality of blades 313A placed by a predetermined interval length on the outer peripheral surface thereof. Here, the plurality of blades 313A is placed along the shaft center 312. The lower blade roll 315 is provided with: a suction hole 315A configured to suck each of the flaps 30 formed by cutting the flap continuum 130; and a stationary blade (not shown) configured to cut the flap continuum 130 with the blade 313A.

The peripheral surface of the lower blade roll 315 is provided with: a first zone Z1 that sucks and holds the flap continuum 130 with a predetermined suction force; and a second zone Z2 that sucks and holds each of the flaps 30 formed by cutting the flap continuum 130, with a suction force stronger than that of the first zone Z1.

When the flap continuum 130 passes through the first zone Z1, while slipping around the peripheral surface of the lower blade roll 315, the flap continuum 130 is sucked by the suction holes 315A formed on the first zone Z1 so as to be held by the peripheral surface of the lower blade roll 315. At this time, the flap continuum 130 is cut between the blade 313A and the stationary blade. Thereby, each of the flaps 30 is formed.

When each of the flaps 30 passes through the second zone Z2, without slipping around the peripheral surface of the lower blade roll 315, the flaps 30 are sucked by the suction holes 315A formed on the second zone Z2 so as to be held by the peripheral surface of the lower blade roll 315. Then, the flaps 30 approach the rear front waistline member continuum 120a by the rotation of the lower blade roll 315 so as to be attached thereon.

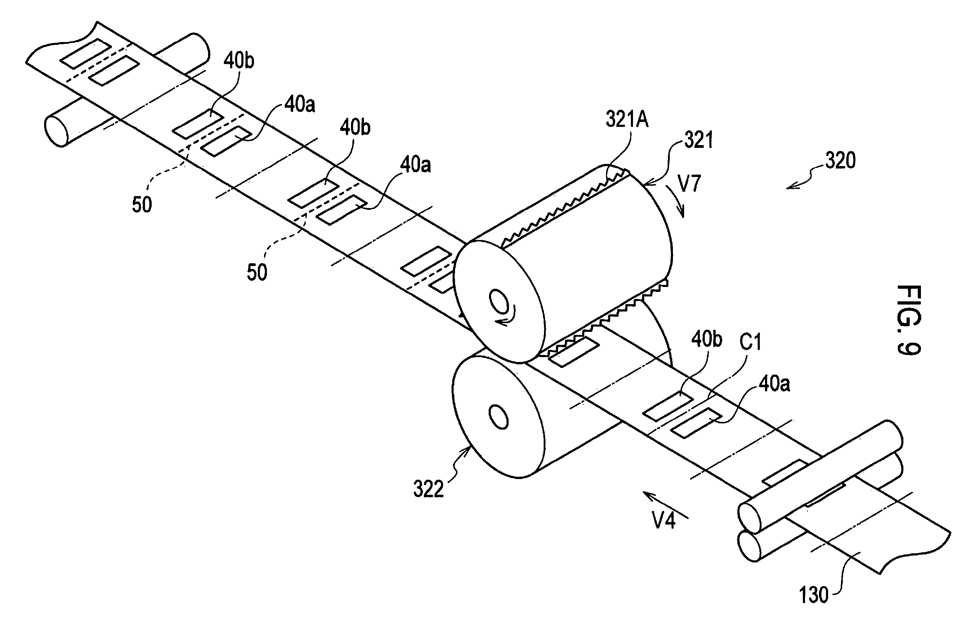

Next, the potential cut line forming structure 320 according to the second embodiment of the present invention will be described by referring to the accompanying drawings. Fig. 9 is a perspective view of the potential cut line forming structure 320 according to the second embodiment of the present invention.

As shown in Fig. 9, as described in the above step S22, the potential cut line forming structure 320 forms a potential cut line 50 along the center line C1. The potential cut line forming structure 320 is provided with: a cutting roll 321 configured to rotate by using a shaft (not shown) as a center; and a nipping roll 322 configured to nip the flap continuum 130 between the cutting roll 231 and the nipping roll 232.

The circumferential velocity (V₇) of the cutting roll 321 is substantially the same as the moving velocity (V₄) of the flap continuum 130. The cutting roll 231 includes a cutting process protrusion 231 A for forming the potential cut line along the center line C1. The cutting process protrusion 231 A is formed in the CD direction of the flap line, that is, the shaft direction of the cutting roll 321.

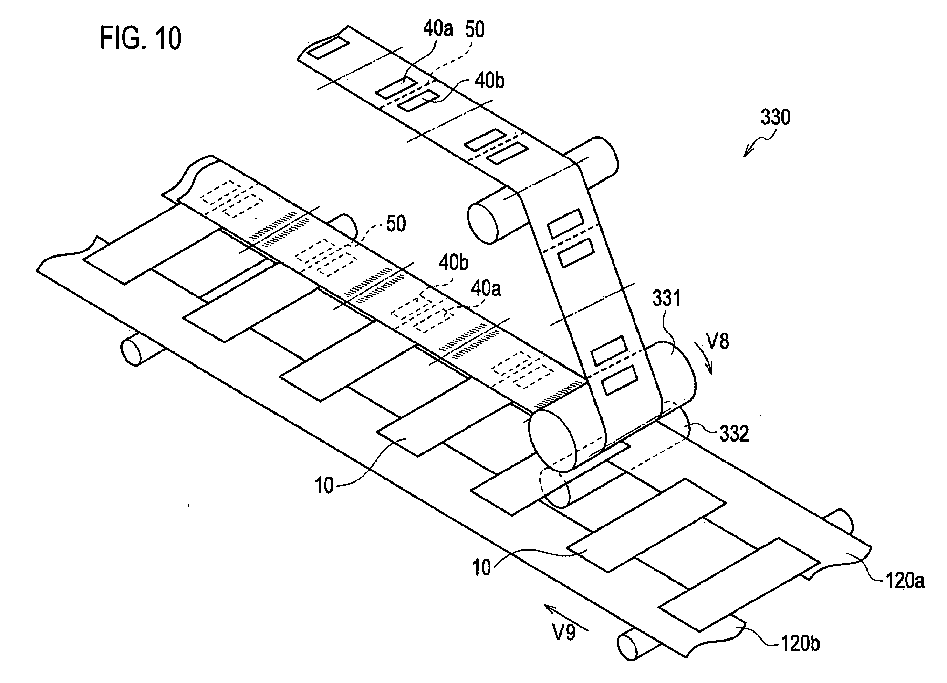

Subsequently, the bonding structure 330 according to the second embodiment of the present invention will be described by referring to the accompanying drawings. Fig. 10 is a perspective view of the bonding structure 330 according to the second embodiment of the present invention.

As shown in Fig. 10, the bonding structure 330 bonds the rear waistline member continuum 120a and each of the flaps 30 on the main body line, so that the center line C1 is orthogonal to the MD direction of the main body line.

The bonding structure 330 is provided with a pair of rollers 331 and 332. Note that, the circumferential velocity (V₈) of the pair of rollers 331 and 332 is substantially the same as the moving velocity (V₄) of the flap continuum 130.

The roller 331 guides the flap continuum 130 to the rear waistline member continuum 120a. Meanwhile, the roller 332 nips the rear waistline member continuum 120a and the flap continuum 130, and faces the roller 331. The pair of rollers 331 and 332 nips the rear waistline member continuum 120a and the flap continuum 130 therebetween, thereby the bonding structure 330 bonds the rear waistline member continuum 120a and the flaps 30.

### [Other Embodiments]

It is a matter of course that orders of performing the steps in the above-described manufacturing method for the absorbent articles is not limited to those described above as long as the absorbent article can be manufactured, and the steps to be performed can be appropriately selected in accordance with the intended purpose.

As described above, the present invention has been described in detail by using the above-described embodiments. However, it is obvious for a person skilled in the art that the present invention is not limited to the embodiments described in this specification. The present invention can be implemented as a modification and an amended embodiment without departing from the content and scope of the present invention which is defined by the description of the scope of claims. Accordingly, the description of the present invention is intended to give description as an example and does not have any meaning to limit the present invention.

### INDUSTRIAL APPLICABILITY

As described above, according to the manufacturing method for absorbent articles, there is provided a manufacturing method for absorbent articles being able to form side flaps that can be easily unfolded and developed by the user, and being able to achieve a continuous transportation of a flap continuum on a flap line without using high level controls.

## Claims

1. A manufacturing method for absorbent articles each including a front waistline member (20b), a rear waistline member (20a), and a crotch member (10), the crotch member connecting the front waistline member and the rear waistline member, the method comprising:
placing latch members (40a, 40b) that can be attached to and locked in predetermined regions, on both sides of a vicinal region of a center line (C1) extending along a moving direction of a flap line or a direction crossing the moving direction of the flap line, on an area each constituting flaps (30) from a flap continuum (130) having a long shape and being continuously transported on the flap line;
forming a potential cut line (50) along the center line;
bonding, on a main body line, a front waistline member continuum (120b) or a rear waistline member continuum (120a) onto the flap continuum (130) or onto the flaps (30), so that the center line is orthogonal to a moving direction of the main body line; the flaps being formed by cutting the flap continuum along a direction crossing the moving direction of the flap line; and
tearing along the potential cut line on the main body line.

2. The manufacturing method for the absorbent articles according to claim 1, wherein,
in the bonding, the flap continuum (130) or each of the flaps (30) is bonded to the front waistline member continuum (120b) or the rear waistline member continuum (120a), so that each of the regions (30A) constituting each of the flaps (30) respectively corresponds to each of the regions (20A) constituting each of the absorbent articles, on the front waistline member continuum (120b) or the rear waistline member continuum (120a).

3. The manufacturing method for the absorbent articles according to claim 1, further including:
forming the flaps (30) on the flap line, by cutting the flap continuum (130) in a direction crossing the moving direction of the flap line.

4. The manufacturing method for the absorbent articles according to claim 1, wherein,
in the tearing, the potential cut line is torn by setting a tension (T1) employed after the bonding on the main body line to be stronger than a tension (T2) employed before the bonding on the main body line.

5. The manufacturing method for the absorbent articles according to claim 1, wherein,
in the tearing, the potential cut line is torn by being pressed with a roller having a protruding portion.

6. The manufacturing method for the absorbent articles according to claim 1, wherein,
in the tearing, the potential cut line is torn by a hammer pressing.

7. The manufacturing method for the absorbent articles according to any one of claims 4 to 6, wherein,
in the tearing, a region including the potential cut line is heat treated to be made into a film.

8. The manufacturing method for the absorbent articles according to claim 1, further comprising:
placing the crotch members (10) so that each of the crotch members is apart from each other in the moving direction of the main body line, between the front waistline member continuum (120b) and the rear waistline member continuum (120a) both having a long shape and being transported continuously on the main body line.

9. The manufacturing method for the absorbent articles according to claim 1, wherein,
in the bonding, the flap continuum (130) or the flaps (30) are placed so as to cover edge portions of the crotch member.

10. A manufacturing apparatus for absorbent articles each including a front waistline member (20b), a rear waistline member (20a), and a crotch member (10), the crotch member connecting the front waistline member and the rear waistline member, the apparatus comprising:
a latch member placing structure (310) configured to place latch members (40a, 40b) that can be attached to and locked in predetermined regions, on both sides of a vicinal region of a center line (C1) extending along a moving direction of a flap line or a direction crossing the moving direction of the flap line, on an area each constituting flaps (30) from a flap continuum (130) having a long shape and being continuously transported on a flap line;
a potential cut line forming structure (320) configured to form a potential cut line (50) along the center line;
a bonding structure (330) configured to bond a front waistline member continuum (120b) or a rear waistline member continuum (120a) onto the flap continuum (130) or onto the flaps (30), so that the center line is orthogonal to a moving direction of a main body line; the flaps being formed by cutting the flap continuum along a direction crossing the moving direction of the flap line; and
a tearing structure (200) configured to tear along the potential cut line on the main body line.

## Patentansprüche

1. Verfahren zur Herstellung von saugfähigen Artikeln, die jeweils ein vorderes Taillenteil (20b), ein hinteres Taillenteil (20a) und ein Schrittteil (10) umfassen, wobei das Schrittteil (10) das vordere Taillenteil mit dem hinteren Taillenteil verbindet, wobei das Verfahren umfasst:
das Auflegen von Verschlussteilen (40a, 40b), die in vorgegebenen Bereichen auf beiden Seiten eines Nachbarbereichs einer Mittellinie (C1), die entlang einer Bewegungsrichtung einer Laschenstraße oder einer die Bewegungsrichtung der Laschenstraße überkreuzenden Richtung verläuft, befestigt und dort verriegelt werden können, auf je eine Fläche, die aus Laschen (30) von einem Laschenkontinuum (130) besteht, das eine längliche Form hat und kontinuierlich auf der Laschenstraße gefördert wird;
das Bilden einer potentiellen Schnittlinie (50) entlang der Mittellinie;
das Verbinden eines vorderen Taillenteilkontinuums (120b) oder eines hinteren Taillenteilkontinuums (120a) mit dem Laschenkontinuum (130) oder mit den Laschen (30) auf einer Hauptkörperstraße, so dass die Mittellinie orthogonal zu einer Bewegungsrichtung der Hauptkörperstraße verläuft, wobei die Laschen durch Schneiden des Laschenkontinuums in einer die Bewegungsrichtung der Laschenstraße überkreuzenden Richtung gebildet werden; und
das Reißen entlang der potentiellen Schnittlinie auf der Hauptkörperstraße.

2. Verfahren zur Herstellung von saugfähigen Artikeln nach Anspruch 1, wobei
beim Verbindungsvorgang das Laschenkontinuum (130) oder die jeweiligen Laschen (30) mit dem vorderen Taillenteilkontinuum (120b) oder dem hinteren Taillenteilkontinuum (120a) so verbunden wird/werden, dass die jeweiligen die Laschen (30) bildenden Bereiche (30A) den jeweiligen die saugfähigen Artikel bildenden Bereichen (20A) am vorderen Taillenteilkontinuum (120b) oder am hinteren Taillenteilkontinuum (120a) entsprechen.

3. Verfahren zur Herstellung von saugfähigen Artikeln nach Anspruch 1, weiter umfassend:
das Bilden der Laschen (30) auf der Laschenstraße durch Schneiden des Laschenkontinuums (130) in einer die Bewegungsrichtung der Laschenstraße überkreuzenden Richtung.

4. Verfahren zur Herstellung von saugfähigen Artikeln nach Anspruch 1, wobei
beim Reißvorgang die potentielle Schnittlinie durch Einstellen einer Spannung (T1) aufgerissen wird, die nach dem Verbinden auf der Hauptkörperstraße zur Anwendung kommt und stärker ist als eine Spannung (T2), die vor dem Verbinden auf der Hauptkörperstraße zur Anwendung kommt.

5. Verfahren zur Herstellung von saugfähigen Artikeln nach Anspruch 1, wobei
beim Reißvorgang die potentielle Schnittlinie durch Drücken mit einer Rolle mit einer hervorstehenden Partie aufgerissen wird.

6. Verfahren zur Herstellung von saugfähigen Artikeln nach Anspruch 1, wobei beim Reißvorgang die potentielle Schnittlinie durch Hammerdruck aufgerissen wird.

7. Verfahren zur Herstellung von saugfähigen Artikeln nach einem der Ansprüche 4 bis 6, wobei
beim Reißvorgang ein die potentielle Schnittlinie umfassender Bereich durch Wärmebehandlung zu einem Film verarbeitet wird.

8. Verfahren zur Herstellung von saugfähigen Artikeln nach Anspruch 1, weiter umfassend:
das Auflegen der Schrittteile (10) auf eine Art und Weise, so dass die Schrittteile jeweils in der Bewegungsrichtung der Hauptkörperstraße voneinander beabstandet sind, zwischen dem vorderen Taillenteilkontinuum (120b) und dem hinteren Taillenteilkontinuum (120a), die jeweils eine längliche Form haben und kontinuierlich auf der Hauptkörperstraße gefördert werden.

9. Verfahren zur Herstellung von saugfähigen Artikeln nach Anspruch 1, wobei
beim Verbindungsvorgang das Laschenkontinuum (130) oder die Laschen (30) so aufgelegt wird/werden, dass die Randpartien des Schrittteils verdeckt werden.

10. Vorrichtung zur Herstellung von saugfähigen Artikeln, die jeweils ein vorderes Taillenteil (20b), ein hinteres Taillenteil (20a) und ein Schrittteil (10) umfassen, wobei das Schrittteil das vordere Taillenteil mit dem hinteren Taillenteil verbindet, wobei die Vorrichtung umfasst:
eine Verschlussteil-Platzierstruktur (310), die zum Platzieren von Verschlussteilen (40a, 40b), die in vorgegebenen Bereichen auf beiden Seiten eines Nachbarbereichs einer Mittellinie (C1), die entlang einer Bewegungsrichtung einer Laschenstraße oder einer die Bewegungsrichtung der Laschenstraße überkreuzenden Richtung verläuft, befestigt und dort verriegelt werden können, auf je eine Fläche ausgelegt ist, die aus Laschen (30) von einem Laschenkontinuum (130) besteht, das eine längliche Form hat und kontinuierlich auf der Laschenstraße gefördert wird;
eine Bildungsstruktur (320) für potentielle Schnittlinien, die zur Bildung einer potentiellen Schnittlinie (50) entlang der Mittellinie ausgelegt ist;
eine Verbindungsstruktur (330), die zur Verbindung eines vorderen Taillenteilkontinuums (120b) oder eines hinteren Taillenteilkontinuums (120a) mit dem Laschenkontinuum (130) oder der Lasche (30) ausgelegt ist, so dass die Mittellinie orthogonal zu einer Bewegungsrichtung einer Hauptkörperstraße verläuft, wobei die Laschen durch Schneiden des Laschenkontinuums in einer die Bewegungsrichtung der Laschenstraße überkreuzenden Richtung gebildet werden; und
eine Reißstruktur (200), die zum Reißen entlang der potentiellen Schnittlinie auf der Hauptkörperstraße ausgelegt ist.

## Revendications

1. Procédé de fabrication pour des articles absorbants comprenant chacun un élément avant au niveau de la taille (20b), un élément arrière au niveau de la taille (20a) et un élément au niveau de l'entrejambe (10), l'élément au niveau de l'entrejambe reliant l'élément avant au niveau de la taille et l'élément arrière au niveau de la taille, le procédé comportant :
l'étape consistant à placer des éléments de maintien (40a, 40b) qui peuvent être attachés sur et verrouillés dans des régions prédéterminées, des deux côtés d'une région vicinale d'une ligne centrale (C1) s'étendant le long d'une direction de mouvement d'une ligne de rabats ou d'une direction croisant la direction de mouvement de la ligne de rabats, sur une zone constituant chacune des rabats (30) à partir d'un continuum de rabats (130) ayant une longue forme et étant continuellement transporté sur la ligne de rabats ;
l'étape consistant à former une ligne de coupe potentielle (50) le long de la ligne centrale ;
l'étape consistant à lier, sur une ligne de corps principaux, un continuum d'éléments avant au niveau de la taille (120b) ou un continuum d'éléments arrière au niveau de la taille (120a) sur le continuum de rabats (130) ou sur les rabats (30), de telle sorte que la ligne centrale est perpendiculaire à une direction de mouvement de la ligne de corps principaux ; les rabats étant formés en coupant le continuum de rabats le long d'une direction croisant la direction de mouvement de la ligne de rabats ; et
l'étape consistant à déchirer le long de la ligne de coupe potentielle sur la ligne de corps principaux.

2. Procédé de fabrication pour les articles absorbants selon la revendication 1, dans lequel,
au cours de l'étape consistant à lier, le continuum de rabats (130) ou chacun des rabats (30) est lié au continuum d'éléments avant au niveau de la taille (120b) ou au continuum d'éléments arrière au niveau de la taille (120a), de telle sorte que chacune des régions (30A) constituant chacun des rabats (30) respectivement correspond à chacune des régions (20A) constituant chacun des articles absorbants, sur le continuum d'éléments avant au niveau de la taille (120b) ou sur le continuum d'éléments arrière au niveau de la taille (120a).

3. Procédé de fabrication pour les articles absorbants selon la revendication 1, comportant par ailleurs :
l'étape consistant à former les rabats (30) sur la ligne de rabats, en coupant le continuum de rabats (130) dans une direction croisant la direction de mouvement de la ligne de rabats.

4. Procédé de fabrication pour les articles absorbants selon la revendication 1, dans lequel,
au cours de l'étape consistant à déchirer, la ligne de coupe potentielle est déchirée en réglant une tension (T1) utilisée après la liaison sur la ligne de corps principaux qui est supérieure à une tension (T2) utilisée avant la liaison sur la ligne de corps principaux.

5. Procédé de fabrication pour les articles absorbants selon la revendication 1, dans lequel,
au cours de l'étape consistant à déchirer, la ligne de coupe potentielle est déchirée en étant comprimée au moyen d'un cylindre ayant une partie faisant saillie.

6. Procédé de fabrication pour les articles absorbants selon la revendication 1, dans lequel,
au cours de l'étape consistant à déchirer, la ligne de coupe potentielle est déchirée par une compression au marteau.

7. Procédé de fabrication pour les articles absorbants selon l'une quelconque des revendications 4 à 6, dans lequel,
au cours de l'étape consistant à déchirer, une région comprenant la ligne de coupe potentielle est traitée à chaud à des fins de transformation en un film.

8. Procédé de fabrication pour les articles absorbants selon la revendication 1, comportant par ailleurs :
l'étape consistant à placer les éléments au niveau de l'entrejambe (10) de telle sorte que chacun des éléments au niveau de l'entrejambe est séparé par rapport à un autre dans la direction de mouvement de la ligne de corps principaux, entre le continuum d'éléments avant au niveau de la taille (120b) et le continuum d'éléments arrière au niveau de la taille (120a) ayant tous les deux une longue forme et étant transportés de manière continue sur la ligne de corps principaux.

9. Procédé de fabrication pour les articles absorbants selon la revendication 1, dans lequel,
au cours de l'étape consistant à lier, le continuum de rabats (130) ou les rabats (30) sont placés de manière à recouvrir des parties de bord de l'élément au niveau de l'entrejambe.

10. Appareil de fabrication pour des articles absorbants comprenant chacun un élément avant au niveau de la taille (20b), un élément arrière au niveau de la taille (20a) et un élément au niveau de l'entrejambe (10), l'élément au niveau de l'entrejambe reliant l'élément avant au niveau de la taille et l'élément arrière au niveau de la taille, l'appareil comportant :
une structure de mise en place d'éléments de maintien (310) configurée pour placer des éléments de maintien (40a, 40b) qui peuvent être attachés sur et verrouillés dans des régions prédéterminées, des deux côtés d'une région vicinale d'une ligne centrale (C1) s'étendant le long d'une direction de mouvement d'une ligne de rabats ou d'une direction croisant la direction de mouvement de la ligne de rabats, sur une zone constituant chacune des rabats (30) à partir d'un continuum de rabats (130) ayant une longue forme et étant continuellement transporté sur la ligne de rabats ;
une structure de formation de ligne de coupe potentielle (320) configurée pour former une ligne de coupe potentielle (50) le long de la ligne centrale ;
une structure de liaison (330) configurée pour lier un continuum d'éléments avant au niveau de la taille (120b) ou un continuum d'éléments arrière au niveau de la taille (120a) sur le continuum de rabats (130) ou sur les rabats (30), de telle sorte que la ligne centrale est perpendiculaire à une direction de mouvement d'une ligne de corps principaux ; les rabats étant formés en coupant le continuum de rabats le long d'une direction croisant la direction de mouvement de la ligne de rabats ; et
une structure de déchirure (200) configurée pour déchirer le long de la ligne de coupe potentielle sur la ligne de corps principaux.
